# EUROPÄISCHE PATENTANMELDUNG

(11) **EP 2 407 180 A1**
(43) Veröffentlichungstag der Anmeldung: **18.01.2012**
(21) Anmeldenummer: 10007348.5
(22) Anmeldetag: 15.07.2010
(51) Int. Cl.: A61L 2/18, A61L 2/22, C02F 1/467

(54) **Verfahren zur Desinfektion von Oberflächen, Vernebelungsvorrichtungen und mobiles Austragsgerät**

(71) Anmelder: ASECA AG, 6314 Unterägeri (CH)
(72) Erfinder: Mathé, Hans-Georg, 78647 Trossingen (DE)
(74) Vertreter: Tergau & Walkenhorst

(57) **Zusammenfassung**

Ein Verfahren zur Desinfektion von Oberflächen soll bei zuverlässigem Desinfektionserfolg eine besonders effektive und bedarfsgerechte Nutzung verfügbarer Umgebungsressourcen bei hoher Flexibilität ermöglichen. Dazu wird erfindungsgemäß dezentral und in räumlicher Nähe zur zu behandelnden Oberfläche ein Desinfektionsmittel durch Zudosierung eines Konzentrats eines Desinfektionswirkstoffes zu einem Brauchwasserstrom erzeugt und über eine Austragseinheit auf die zu behandelnde Oberfläche aufgetragen.

## Beschreibung

Die Erfindung betrifft ein Verfahren zur Desinfektion von Oberflächen.,Sie bezieht sich weiter auf eine Vernebelungsvorrichtung für ein Desinfektionsmittel und auf ein mobiles Austragsgerät zur Ausbringung eines Desinfektionsmittels auf eine zu behandelnde Oberfläche, insbesondere zur Durchführung des Verfahrens.

Die zuverlässige Desinfektion von Oberfläche ist in einer Vielzahl von Anwendungsgebieten von großer Bedeutung. Dabei wird ein geeignet ausgewähltes Desinfektionsmittel auf die zu behandelnde Oberfläche aufgebracht, um dort eventuell vorhandene Keime oder dergleichen abzutöten. Üblicherweise steht dabei, gerade im Hinblick auf möglicherweise vorgegebene Randbedingungen wie Sterilität oder dergleichen der zuverlässige Desinfektionserfolg, also eine restlose Beseitigung der Keime oder dergleichen, absolut im Vordergrund der Desinfektionsbehandlung. Aus diesen Gründen werden für eine effektive Desinfektion vergleichsweise aggressive Desinfektionsmittel verwendet, so z.B. Ozon oder Formaldehyd. Diese wirken jedoch nicht nur keimtötend, sondern auch toxisch und können beispielsweise Allergien, Haut-, Atemwegs- oder Augenreizungen verursachen.

Sowohl im Hinblick auf diese Materialeigenschaften üblicher Desinfektionsmittel als auch im Hinblick auf üblicherweise für die Desinfektion vorhandene Umgebungs- oder Systemressourcen ist dabei aber ein flexobler und bedarfsgerechter Einsatz der Desinfektionsmittel nur begrenzt möglich.

Der Erfindung liegt daher die Aufgabe zu Grunde, ein Verfahren zur Desinfektion von Oberflächen anzugeben, das bei zuverlässigem Desinfektionserfolg eine besonders effektive und bedarfsgerechte Nutzung verfügbarer Umgebungsressourcen bei hoher Flexibilität ermöglicht. Des Weiteren sollen eine für die Durchführung des Verfahrens besonders geeignete Vernebelungsvorrichtung für ein Desinfektionsmittel sowie ein für die Durchführung des Verfahrens besonders geeignetes Austragsgerät angegeben werden.

Bezüglich des Verfahrens wird diese Aufgabe erfindungsgemäß gelöst, indem dezentral und in räumlicher Nähe zur zu behandelnden Oberfläche das Desinfektionsmittel durch Zudosierung eines Konzentrats eines Desinfektionswirkstoffes zu einem Brauchwasserstrom erzeugt und über eine Austragseinheit auf die zu behandelnde Oberfläche aufgetragen wird.

Die Erfindung geht dabei von der Überlegung aus, dass ein besonders bedarfsgerechter und damit ressourcenschonender Einsatz des Desinfektionsmittels erreichbar ist, indem unter weitgehendem Rückgriff auf lokal verfügbare Ressourcen das eigentlich auszubringende Desinfektionsmittel dezentral und unmittelbar am Einsatzort und auch unmittelbar vor dem eigentlichen Einsatz hergestellt wird. Dies ist ermöglicht, indem zur Herstellung des Desinfektionsmittels auf eine weithin ohnehin verfügbare Ressource, nämlich auf im Rahmen eines üblicherweise vorhandenen Brauchwasser-Versorgungsnetzes verfügbares Brauchwasser, zurückgegriffen und das Desinfektionsmittel durch Zumischung eines Konzentrats eines Desinfektionswirkstoffes zu einem Brauchwasserstrom erzeugt wird.

Um dabei eine an sich unerwünschte Belastung der Umwelt oder der Umgebung durch aggressive Wirkstoffe zuverlässig zu vermeiden, wird in besonders vorteilhafter Ausgestaltung als Konzentrat zur Erzeugung des Desinfektionsmittels eine elektrochemisch aktivierte Lösung verwendet, welche durch Elektrolyse von solehaltigem Wasser in einem eine Mehrzahl von Elektrolysezellen umfassenden Elektrolysemodul erzeugt ist, bei dem jede Elektrolysezelle jeweils einen ersten Elektrodenraum und einen von diesem durch eine Membran getrennten zweiten Elektrodenraum umfasst, wobei der Elektrolyt den ersten Elektrodenräumen der Elektrolysezellen parallel und den zweiten Elektrodenräumen der Elektrolysezellen in Reihenschaltung zugeführt wird.

Eine wichtige Kennzahl für die Nutzbarkeit der elektrochemisch aktivierten Salzlösungen in derartigen Anwendungen ist der Gehalt an freien Chlorinen sowie der pH-Wert. Für eine besonders hohe bakteriozide oder antibakterielle Wirkung ist hierbei ein möglichst hoher Gehalt an freiem Chlorin wünschenswert, wobei aber gerade im Hinblick auf Verwendungen als Desinfektionsmittel für die Raumdesinfektion, für die Oberflächendesinfektion oder für medizinische Zwecke eine besonders gute medizinische Verträglichkeit wünschenswert ist. Wie sich überraschenderweise herausgestellt hat, ist ein Desinfektionswirkstoff mit derartigen Eigenschaften durch die Bereitstellung einer elektrochemisch aktivierten Lösung auf die genannte Weise herstellbar. Dieser ist somit gerade im Hinblick auf seine besonders gute biologische Kompatibilität für die Verwendung bei einer dezentralen Herstellung des Desinfektionsmittels besonders gut geeignet.

Das bei zur Erzeugung des Desinfektionsmittels verwendete Elektrolysemodul umfasst vorzugsweise mehrere Elektrolysezellen mit jeweils zumindest zwei Elektrodenräumen, nämlich einen zum elektrischen Anschluss an den ,,+"-Pol einer anzulegenden Elektrolysespannung vorgesehenen Anodenraum und einem zum elektrischen Anschluss an den ,,-" -Pol der Elektrolysespannung vorgesehenen Kathodenraum. Die Elektrodenräume der ersten Polarität, also die Kathodenräume oder alternativ auch die Anodenräume, sollen dabei medienseitig, also bzgl. der Beaufschlagung mit Elektrolyt und auch bzgl. der Abführung des ,,verbrauchten" Elektrolyts, parallel geschaltet sein. Dabei werden aus einem gemeinsamen Versorgungssystem die jeweiligen ersten Elektrodenräume der einzelnen Elektrolysezellen aus einer sich verzweigenden gemeinsamen Versorgungsleitung bedient, und der nach dem Durchlauf durch die jeweiligen ersten Elektrodenräume noch verbleibende Elektrolyt wird anschließend in einem gemeinsamen Abführsystem aus den ersten Elektrodenräumen abgeführt. Im Gegensatz zu dieser parallelen Medienstromführung durch die Elektrodenräume der ersten elektrischen Polarität ist seitens der zweiten, dem elektrisch ,,anderen" Pol zugeordneten Elektrodenräume aber in der Art einer mehrstufigen oder kaskadierten Verschaltung vorgesehen, dass diese Elektrodenräume der Elektrolysezellen vom Elektrolyt sequentiell oder hintereinander durchströmt werden sollen. Somit erfährt der Elektrolyt in der Art einer kaskadierten Behandlung stufenweise in den einzelnen Elektrolysezellen hintereinander mehrfache Behandlungsschritte, so dass eine mehrstufige und damit besonders weit gehende Anreicherung von Ionen im Elektrolyten erreichbar ist.

Die kaskadenartige Durchströmung einer Mehrzahl von hintereinandergeschalteten Elektrodenräumen kann dabei anodenseitig (bei paralleler Durchströmung der Kathodenräume), kathodenseitig (bei paralleler Durchströmung der Anodenräume) oder auch in serieller Kombination derartiger Verschaltungsstufen vorgesehen sein. Zur Herstellung einer hochgradig wirksamen Lösung, charakterisiert durch einen besonders hohen Gehalt an freien Chlorinen, ist in besonders vorteilhafter Ausgestaltung die medienseitige Parallelschaltung der Kathodenräume der Elektrolysezellen bei medienseitiger Reihenschaltung der Anodenräume der Elektrolysezellen vorgesehen. Durch eine derartige kaskadierte Behandlung gerade des Anolyten kann eine besonders weitgehende Anreicherung und Aufkonzentration von Chloridionen im Anolyten erreicht werden, so dass gerade im Hinblick auf beispielsweise gewünschte Desinfektionseigenschaften besonders hochwertiger Anolyt hergestellt werden kann. Alternativ ist durch umgekehrte Verschaltung (kaskadenartige Serienschaltung der Kathodenräume bei paralleler Verschaltung der Anodensäure) die Bereitstellung besonders hochwertigen Katholyts mit günstigen Wascheigenschaften möglich.

Wie sich überraschenderweise herausgestellt hat, kann gerade auf diese Weise im Anolyten nach dem Durchlaufen von mehreren, insbesondere von mindestens zwei, Behandlungsstufen ein besonders hoher Gehalt an freiem Chlorin von bis zu 2.000 mg/L erreicht werden, wobei der solchermaßen hergestellte Anolyt zudem auch noch eine besonders hohe Lagerbeständigkeit aufweist. Die nachfolgenden Erläuterungen besonders bevorzugter Ausführungsformen der Erfindung erfolgen daher anhand der Variante einer medienseitigen Parallelschaltung der Kathodenräume bei medienseitiger Reihenschaltung der Anodenräume; analoge Vorteile sind aber auch bei umgekehrter Verschaltung, also Parallelschaltung der Anodenräume in Kombination mit Reihenschaltung der Kathodenräume, erreichbar.

Wie sich weiterhin in überraschender Weise herausgestellt hat, sind besonders günstige Materialeigenschaften des Anolyten hinsichtlich des Gehalts an freiem Chlorin und auch hinsichtlich der Lagerbeständigkeit erreichbar, indem in vorteilhafter Ausgestaltung aus den ersten Elektrodenräumen der Elektrolysezellen abströmender Elektrolyt dem zweiten Elektrodenraum der in Strömungsrichtung des Elektrolyten gesehen ersten Elektrolysezelle zugeführt wird. Mit anderen Worten: Dieser besonders bevorzugten medienseitigen Verschaltung führen die an die Kathodenräume der Elektrolysezellen angeschlossenen Abströmleitungen vorzugsweise zu einem Sammelpunkt, an dem sie in eine gemeinsame Leitung einmünden. Diese ist ausgangsseitig an den in Strömungsrichtung des Anolyten gesehen ersten Anodenraum der von den Elektrolysezellen anodenseitig gebildeten Kaskade angeschlossen. Dieser Schaltung wird das aus den Kathodenräumen abströmende Katholyt somit als Anolyt der mehrstufigen Kaskadenschaltung aus Anodenräumen zugeführt. Hierdurch kann unter anderem die ,,Abfallmenge" des in diesem Fall nicht erwünschten Katholyts besonders gering gehalten werden.

In weiterer vorteilhafter Ausgestaltung wird dabei lediglich ein Teilstrom des aus den ersten Elektrodenräumen der Elektrolysezellen abströmenden Elektrolyts dem zweiten Elektrodenraum der in Strömungsrichtung des Elektrolyten gesehen ersten Elektrolysezelle zugeführt. Wie sich überraschenderweise herausgestellt hat, können gerade durch geeignete Einstellung und Veränderung des Verzweigungsverhältnisses in diesem Bereich, also durch gezielte Einstellung des Anteils am Gesamtstrom des aus den Kathodenräumen abströmenden Katholyts, der als Anolyt der weiteren Behandlung zugeführt wird, gezielt wesentliche Parameter des entstehenden Endprodukts, insbesondere Gehalt an freiem Chlorin, der pH-Wert und/oder die Lagerbeständigkeit, beeinflusst werden. Insbesondere kann durch geeignete Einstellung des Verzweigungsverhältnisses die Durchlaufrate und damit die Verweil- und Reaktionszeit des Anolyten in Relation zum Katholyten verändert werden, so dass eine besonders gezielte Aufkonzentration der Ionen erreichbar ist. Des Weiteren ist dadurch bedarfsweise eine Einstellung der Druckverhältnisse und/oder der Strömungsgeschwindigkeiten möglich.

Zur Herstellung der elektrochemisch aktivierten Salzlösung wird der im Elektrolysemodul vorteilhafterweise ein mit Salzlösung oder Sole versetzter Medienstrom aus enthärtetem Wasser zugeleitet. Die Solekonzentration im Wasserstrom kann dabei vorzugsweise vergleichsweise gering gehalten sein, beispielsweise kann ein 5% - Anteil (Pharma-Aseca z.B. 9% = isotonisch, Lebensmittelbereich < 5%) an gesättigter Sole (diese umfasst einen Salzgehalt Na⁺ CL- 359 g/l) im Wasser für Anwendungen im Lebensmittelbereich oder ein 9%-Anteil (entspricht einer isotonischen Konzentration) für Anwendungen im pharmazeutischen Bereich vorgesehen sein. Zur Einspeisung zur Dosierung der Sole in den Wasserstrom ist vorteilhafterweise eine Venturidüse vorgesehen, die in die den Kathodenräumen vorgeschaltete Zuströmleitung geschaltet und eingangsseitig mit einem Solebehälter verbunden ist. Zur Zuführung eines geeigneten Grundmediums ist zudem in weiterer vorteilhafter Ausgestaltung eine Wasserenthärterstation vorgesehen, über die das enthärtete Wasser zugeführt wird.

Um bei vergleichsweise hohem Durchsatz einen besonders hohen Wirkungsgrad bei der Erzeugung der elektrochemisch aktivierten Salzlösung im Elektrolysemodul zu gewährleisten, ist vorteilhafterweise die für die Ionenwanderung der Bestandteile durch die Membran hindurch verfügbar gehaltene aktive Oberfläche besonders groß. Um dies zu ermöglichen und gezielt zu begünstigen, wird die Anlage in vorteilhafter Ausgestaltung gezielt für eine Minimierung oder Verhinderung einer Verblockung der Elektrodenflächen und/oder Membranflächen durch in den Medienströmen mitgeführte Gasanteile oder -bläschen ausgestaltet. Im Hinblick auf den kathodenseitig in Folge des Elektrolyseprozesses entstehenden Wasserstoff, der in unerwünschter Weise zur Bildung derartiger Gasbläschen beitragen könnte, wird in vorteilhafter Ausgestaltung aus den als Kathodenräume vorgesehene Elektrodenräumen jeweils Gas mittels eines Entgasungsmoduls abgeführt. Dieses umfasst zweckmäßigerweise eine an den Kathodenraum angeschlossene Entgasungsleitung und ggf. darüber hinaus noch geeignete, im Kathodenraum angeordnete Mittel zur Förderung des Gasaustrags aus dem Katholyten, beispielsweise Verwirbler.

In konstruktiver Hinsicht sind grundsätzlich verschiedene Möglichkeiten zur Ausgestaltung des Elektrolysemoduls und der dieses bildenden Elektrolysezellen möglich. Insbesondere könnten die Elektrolysezellen als so genannte Platten- oder Membranelektrolyseure unter Verwendung von zwischen geeigneten Gehäuse- oder Elektrodenplatten angeordneten keramischen oder kunststoffbasierten Plattenmembranen ausgelegt sein. Eine besonders kompakte Bauweise bei besonders hoher betrieblicher Sicherheit ist aber erreichbar, indem das Elektrolysemodul in vorteilhafter Ausgestaltung in Hohlzylinderbauweise angefertigt ist. Dabei ist vorteilhafterweise ein die Anode der jeweiligen Elektrolysezelle bildender Hohlzylinder konzentrisch von einer Membran und diese wiederum konzentrisch von einem die Kathode bildenden Hohlzylinder umgeben.

Um dabei einen besonders hohen Mediendurchsatz und eine besonders hohe Wirksamkeit bei der Erzeugung der elektrochemisch aktivierten Salzlösung zu ermöglichen, ist der jeweils die Anode bildende Hohlzylinder dabei vorteilhafterweise mit einer Anzahl von Durchströmöffnungen versehen. Die Durchströmöffnungen sind dabei zweckmäßigerweise derart ausgestaltet, dass sie einerseits zur Vergrößerung der aktiven Anodenoberfläche (insbesondere durch Medienkommunikation zwischen Innen- und Außenraum der Anode) beitragen und andererseits beim Durchströmen des Mediums Verwirbelungen in diesem bewirken. Durch diese Verwirbelungen ist einerseits die Durchmischung des Mediums und damit die Homogenität der erzeugten Ionen und Partikel im Medium und andererseits die Benetzung der aktiven Oberfläche mit ,,unverbrauchtem" Medium besonders begünstigt.

Bei der Verwendung von auf die genannte Weise hergestelltem Wirkstoff als Konzentrat für die dezentrale Erzeugung eines Desinfektionsmittels wird das Konzentrat dem Brauchwasser vorteilhafterweise über eine Venturidüse zudosiert. Damit ist auf besonders einfache Weise eine zuverlässige Durchmischung der Komponenten sichergestellt, wobei das Beimischungsverhältnis und damit der Anteil des Konzentrats im Brauchwasserstrom auf besonders einfache Weise durch Nachführung des Wasserdrucks und/oder der Durchflussmenge des Brauchwassers geeignet eingestellt werden kann. Damit ist insbesondere auch während der Ausbringung des Desinfektionsmittels in der Art einer aktiven Nachführung die situationsangepasste Veränderung der Dosierung möglich.

Hinsichtlich der Verneblung des so erzeugten besonders wirksamen Desinfektionsmittels ist eine besonders effektive Raumdesinfektion möglich, indem vorteilhafterweise bei der Verneblung mikroskopische Tröpfchen mit einer Größe von 5-50 Mikron erzeugt werden. Derartig kleine Tröpfchen bilden einen schwebfähigen Nebel, d.h. sie weisen eine Sinkgeschwindigkeit von unter zwei Meter pro Stunde auf. Damit wird einerseits durch die große Reaktionsfläche der Aufwand an Desinfektionsmittel auf ein Minimum reduziert, so dass dieser ca. einen Liter Desinfektionswirkstoff auf 1000 Quadratmeter behandeltes Luftvolumen betragen kann. Andererseits wird eine Desinfektion nicht nur der Oberflächen, auf denen sich der Wirkstoff fein verteilt, sondern auch der Raumluft selbst erreicht. Weiterhin werden nicht nur Geruchspartikel in der Luft neutralisiert, die kleinen Nebeltröpfchen diffundieren auch bis in poröse Materialien hinein und neutralisieren dort alle Geruchsquellen.

Durch die gute Verträglichkeit des so erzeugten Desinfektionsmittels ist es möglich, anstatt einer temporären Desinfektion auch eine permanente Raumluftentkeimung durchzuführen. Dazu wird das Verfahren in vorteilhafter Ausgestaltung in Anwesenheit von Menschen oder Tieren durchgeführt. Gerade in Arztpraxen oder Krankenhäusern ist das Ansteckungsrisiko für Patienten und Mitarbeiter extrem hoch. Mit einer kontinuierlichen Raumluftentkeimung wird dieses Risiko auf ein Minimum reduziert. Darüber hinaus eignet sich das Verfahren hervorragend für alle Räumlichkeiten, in den eine hohe Anzahl von Menschen verkehren, wie z.B. Büroräume, Arztpraxen, Krankenhäuser, Ämter, Wartezimmer usw. In Firmen mit großen Büroeinheiten kann so z.B. der Krankenstand deutlich reduziert werden, da Keime und Bakterien kontinuierlich abgetötet werden und so die Gefahr der Ansteckung (z.B. bei Erkältungen) untereinander deutlich geringer ist als bisher.

Vorteilhafterweise wird ein derartiges Verfahren durch eine Vernebelungsvorrichtung mit einem Desinfektionsmittelbehälter und einer Vernebelungsdüse ausgeführt. Dadurch ist eine automatische permanente oder temporäre Desinfektion von Räumen möglich.

In vorteilhafter Ausgestaltung ist die Vernebelungsdüse dabei aus einem Kunststoff gefertigt. Dadurch wird eine Beschädigung der Vernebelungsdüse durch Korrosion durch das Desinfektionsmittel verhindert, wodurch eine insgesamt höhere Lebensdauer der Vernebelungsvorrichtung erreicht wird.

Bezüglich des Austragsgeräts wird die genannte Aufgabe gelöst mit einem Vorratsbehälter für das Konzentrat des Desinfektionswirkstoffes, mit einem Anschlusssystem zur Verbindung mit einem Brauchwasser-Versorgungsnetz, und mit einer ausgangsseitig mit einer Austragseinheit und eingangsseitig sowohl mit dem Vorratsbehälter als auch mit dem Anschlusssystem verbundenen Dosiereinheit. Das Austragsgerät ist dabei vorzugsweise mobil ausgelegt, so dass ein flexibler Einsatz in unmittelbarer örtlicher Nähe der zu behandelnden Oberfläche und damit eine dezentrale Erzeugung des Desinfektionsmittels ,,vor Ort" durch Zudosierung des Konzentrats in den Brauchwasserstrom ermöglicht ist.

Vorteilhafterweise umfasst die Dosiereinheit eine Venturidüse, über die das Konzentrat in das Brauchwasser eingespeist werden kann. Gerade in Kombination mit einer in alternativer oder zusätzlicher vorteilhafter Weiterbildung ebenfalls vorgesehenen, in den Brauchwasserstrang des Austragsgeräts geschalteten Förderpumpe kann dabei durch geeignete Einstellung der Medienströme zur Venturidüse hin und/oder durch geeignete Einstellung von Wasserdruck oder Durchflussmenge im Wasserstrang die Dosiermenge des Konzentrats im Brauchwasser zuverlässig eingestellt und bedarfsweise auch nachgeführt werden.

Die Austragseinheit ist zweckmäßigerweise als Sprühlanze ausgeführt.

Die mit der Erfindung erzielten Vorteile bestehen insbesondere darin, dass durch die dezentrale Erzeugung des auf die zu behandelnde Oberfläche aufzubringenden Desinfektionsmittels unmittelbar vor Ort, also in unmittelbarer räumlicher Nähe der zu behandelnden Oberfläche, eine besonders bedarfsgerechte und flexible Bereitstellung des Desinfektionsmittels ermöglicht ist. Gerade unter Verwendung üblicherweise vorhandener Ressourcen wie dem in einem Brauchwasser-Versorgungsnetz bereitgehaltenen Brauchwasser ist in logistischer Hinsicht lediglich der Transport vergleichsweise geringer Mengen des Konzentrates des Desinfektionswirkstoffs erforderlich. Dabei kann auf das mobile Austragsgerät zurückgegriffen werden, in dessen Dosiereinheit das über das Anschlusssystem aus dem Brauchwasser-Versorgungsnetz zugeführte Brauchwasser in der geeigneten und gewünschten Dosierung mit dem Konzentrat versetzt werden kann. Durch die Verwendung der Venturidüse in der Dosiereinheit ist dabei die Zudosierung des Konzentrats in das Brauchwasser auf besonders einfache Weise und zuverlässig möglich, wobei die Dosiermenge auf besonders einfache Weise und flexibel eingestellt werden kann.

Durch die Verwendung der elektrochemisch aktivierten Lösung als Konzentrat zur Erzeugung des Desinfikationsmittels ist darüberhinaus ein vergleichsweise hochwirksames Desinfektionsmittel bereitstellbar, wobei auf Grund der Materialeigenschaften des Desinfektionswirkstoffes eine hohe Umweltverträglichkeit gewährleistet ist. Insbesondere kann bei der Herstellung des Desinfektionswirkstoffes durch die Kombination einer parallelen Medienstromführung bei der Bespeisung der ersten Elektrodenräume, vorzugsweise der Kathodenräume, mit einer kaskadenartigen, mehrstufigen Behandlung des Elektrolyten durch Hintereinanderschaltung der zweiten Elektrodenräume, vorzugsweise der Anodenräume, mit besonders hoher Zuverlässigkeit und vergleichsweisen hohem Wirkungsgrad und hoher Durchsatzrate als Anolyt eine elektrochemisch aktivierte Lösung hergestellt und als Grundstoff für das Desinfektionsmittel bereitgestellt werden, die sich gerade hinsichtlich ihrer charakteristischen Eigenschaften wie freiem Chloringehalt und Verträglichkeit deutlich von bisher bekannten Produkten abhebt. Insbesondere kann die elektrochemisch aktivierte Lösung derart hergestellt werden, dass bei einem pH-Wert von etwa 7,6 ein besonders hoher Gehalt an freiem Chlorin, insbesondere von mehr als 500 mg/l oder sogar mehr als 2000 mg/l, bei einem Redoxpotenzial von 700 mV bis 1200 mV, vorzugsweise 800 mV bis 900 mV, erreichbar ist. Durch die gute Verträglichkeit des Desinfektionsmittels ist insbesondere eine zuverlässige Desinfektion der Oberflächen in im Wesentlichen einem Arbeitsgang und ohne Berücksichtigung von Einwirkzeiten oder dergleichen möglich. Die behandelte Fläche ist somit gerade auf Grund der hohen Verträglichkeit des Desinfektionswirkstoffs sofort nach der Behandlung wieder benutzbar, ohne dass chemische Rückstände anfallen. Eine hohe Bio-Verträglichkeit ist damit gegeben. Die Desinfektion der Oberflächen kann dabei ohne Schutzkleidung für das Bedienpersonal vorgenommen werden, ohne dass besondere Sicherungsvorkehrungen oder dergleichen bei Lagerung, Transport oder dergleichen getroffen werden müssten.

Zudem ist gerade durch den Rückgriff auf die elektrochemisch aktivierte Lösung als Desinfektionswirkstoff die Zudosierung des Konzentrats in einen nicht vorgeheizten Kaltwasser-Brauchwasserstrom möglich, so dass der Energieaufwand bei der Herstellung des Desinfektionsmittels besonders gering gehalten werden kann.

Ein Ausführungsbeispiel der Erfindung wird anhand einer Zeichnung näher erläutert. Darin zeigen:
- FIG. 1: eine Anlage zur Erzeugung einer elektrochemisch aktivierten Salzlösung als Desinfektionsmittel durch Elektrolyse,
- FIG. 2: eine alternative Ausführungsform der Anlage nach FIG 1,
- FIG. 3: eine Verneblungsvorrichtung, und
- FIG 4,5: jeweils eine mobile Austragseinheit für ein Desinfektionsmittel.

Gleiche Teile sind in allen Figuren mit denselben Bezugszeichen versehen.

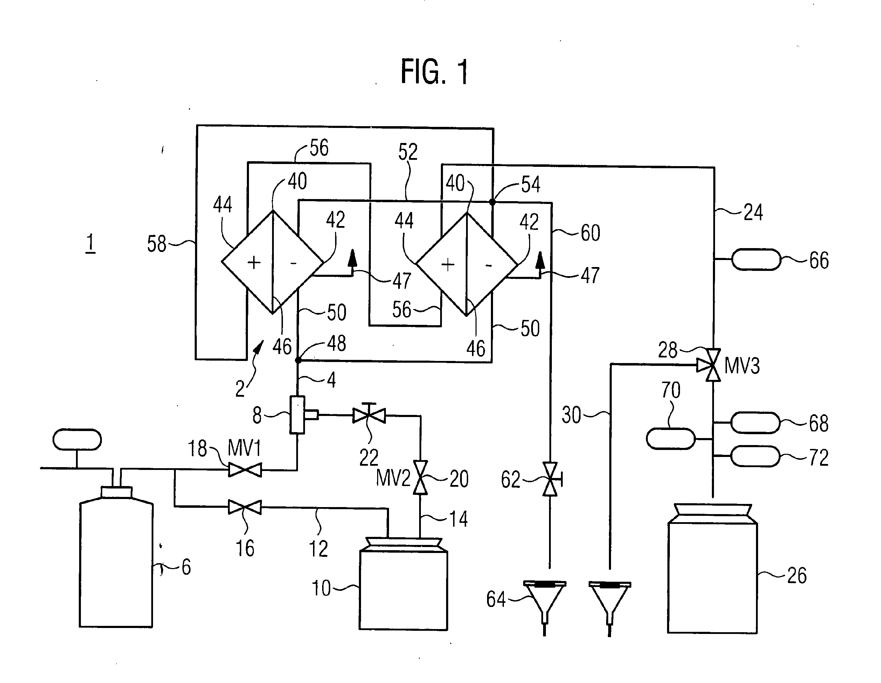

Die Anlage 1 gemäß Figur 1 ist zur Erzeugung einer elektrochemisch aktivierten Salzlösung durch Elektrolyse vorgesehen. Dazu umfasst die Anlage 1 ein Elektrolysemodul 2, dem eingangsseitig über eine Versorgungsleitung 4 ein Elektrolysemedium zuführbar ist. Als Elektrolysemedium ist dabei mit Sole oder einer wässrigen Salzlösung versetztes enthärtetes Wasser vorgesehen. Dazu ist die Zuströmleitung 4 eingangsseitig mit einer Wasserenthärterstation 6 verbunden. Zur Zudosierung der Sole in das enthärtete Wasser ist in die Zuströmleitung 4 eine Venturidüse 8 geschaltet, die ihrerseits eingangsseitig mit einem Solebehälter 10 verbunden ist. Von der Zuführungsleitung 4 zweigt zudem nach der Wasserenthärtestation 6 eine Ablaufleitung 12 ab, über die während einer Inbetriebnahmephase der Anlage 1 der Wasserstrom aus der Wasserenthärterstation 6 unter Umgehung des Elektrolysemoduls 2 der nachfolgenden Komponenten in den Solebehälter 10 abführbar ist. Zur Umschaltung zwischen diesen Betriebszuständen mit dem Dauerbetriebszustand sowie zur dosierten Einspeisung einer vorgebbaren Solemenge in das Elektrolysemedium sind in die Zuführleitung 4, die Ablaufleitung 12 sowie die in die Venturidüse 8 mündende Soleeinspeiseleitung 14 geeignete Ventile 16, 18, 20 und zusätzlich in die Einspeiseleitung 14 ein Drosselventil 20 geschaltet.

Ausgangsseitig, zur Abführung der im Elektrolysemodul 2 hergestellten elektrochemisch aktivierten Salzlösung, ist an das Elektrolysemodul 2 eine Abströmleitung 24 angeschlossen, die ausgangsseitig in einen Vorratsbehälter 26 für die hergestellte Salzlösung oder den Anolyten mündet. Zur vorübergehenden Umgehung des Vorratsbehälters 26 während der Inbetriebnahmephase der Anlage 1 ist in die Ablaufleitung 24 zudem ein Multiwegeventil 28 geschaltet, dessen zweiter Ausgang an eine Abflussleitung 30 angeschlossen ist.

Die Anlage 1 ist zur Herstellung einer elektrochemisch aktivierten Salzlösung mit gerade zur Verwendung als Desinfektionsmittel oder antibakteriellem Wirkstoff in beispielsweise medizinischen oder pharmazeutischen Anwendungen besonders günstigen Eigenschaften, insbesondere mit einem besonders hohen Gehalt an freiem Chlorin von vorzugsweise mehr als 500 mg/L und bis zu 2.000 mg/L bei hoher Lagerfähigkeit, ausgelegt. Um dies zu ermöglichen, ist das Elektrolysemodul 2 mehrkomponentig aufgebaut und umfasst eine Mehrzahl von Elektrolysezellen 40, von denen im Ausführungsbeispiel lediglich zwei dargestellt sind; selbstverständlich können analog zu den folgenden Ausführungen aber auch noch weitere Elektrolysezellen 40 vorgesehen sein.

Jede Elektrolysezelle 40 umfasst jeweils einen ersten Elektrodenraum bildenden Kathodenraum 42 und einen zweiten Elektrodenraum bildenden Anodenraum 44, die jeweils durch eine Membran 46 voneinander getrennt sind. Das Anlegen einer elektrischen Spannung zwischen einer den jeweiligen Anodenraum 44 begrenzenden Anode einerseits und einen den jeweiligen Kathodenraum 42 begrenzenden Kathode andererseits bewirkt sodann eine lonenwanderung über die zwischenliegende Membran 46 hinweg, so dass eine zumindest teilweise Dissoziation des im Elektrolysemedium enthaltenen Wassers sowie eine zumindest teilweise Dissoziation der in Form von Sole mitgeführten Salzanteile auftritt. Auf Grund der elektrischen Ladung der Ionen in diesen Materialien führt dieser lonenwanderungsprozess in Folge der angelegten elektrischen Spannung zu einer Anreicherung von Cl⁻ und OH- im jeweiligen Anodenraum 44 und zu einer Anreicherung von H⁺ und Na⁺ im jeweiligen Kathodenraum 42. Zur bedarfsweisen Abführung von demzufolge im jeweiligen Kathodenraum 42 oder ersten Elektrodenraum angereichertem Wasserstoffgas ist der Kathodenraum 42 jeweils mit einer Entgasungsleitung 47 verbunden. Diese bildet, ggf. in Kombination mit im Kathodenraum 42 angeordneten Mitteln zur Bläschenerzeugung oder Gasabscheidung wie beispielsweise Verwirbler oder dergleichen, ein Entgasungsmodul für den jeweiligen Kathodenraum 42.

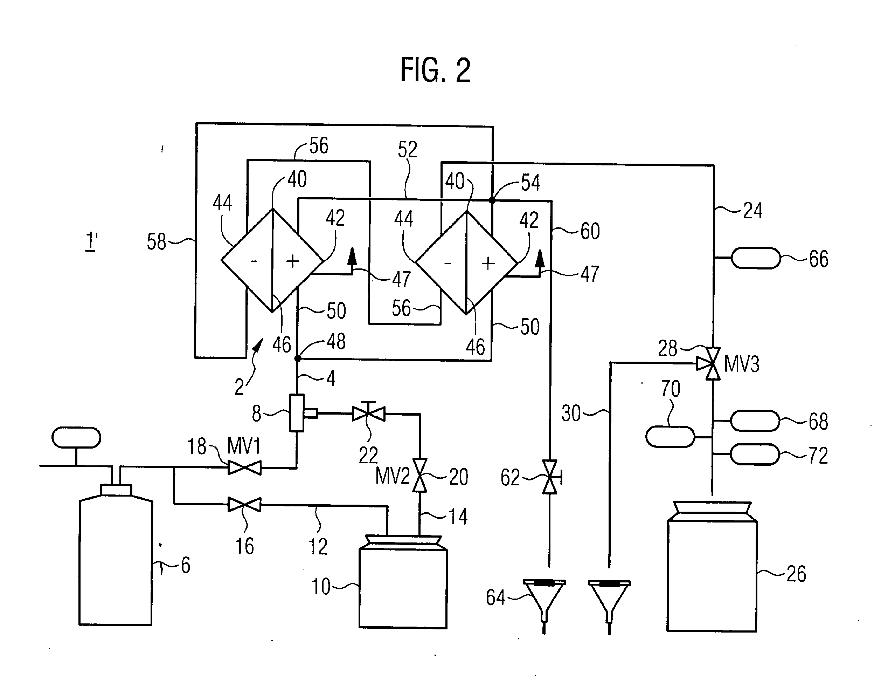

Die gewünschten, qualitativ hochwertigen Eigenschaften der elektrochemisch aktivierten Salzlösung werden in der Anlage 1 insbesondere durch eine spezifische Führung der Medienströme im Elektrolysemodul 2 erreicht. Dabei ist in der Anlage 1 gemäß dem Ausführungsbeispiel, die gezielt auf die Bereitstellung von elektrochemisch hochgradig aktiviertem Anolyt ausgerichtet ist, eine kathodenseitig im Wesentlichen parallele Medienstromführung mit einer anodenseitig im Wesentlichen seriellen Medienstromführung kombiniert. Alternativ oder bei abweichendem Auslegungsziel kann auch eine anodenseitig im Wesentlichen parallele mit einer kathodenseitig im Wesentlichen seriellen Medienstromführung kombiniert sein, wie dies im alternativen Ausführungsbeispiel für die Anlage 1' gemäß FIG. 2 dargestellt ist. Darüberhinaus können auch noch weiterführende Kombinationen dieser medienseitigen Verschaltungen vorgesehen sein.

Im Einzelnen ist dabei in der Anlage 1 im Ausführungsbeispiel in der Zuströmleitung 4 ein Verzweigungspunkt 48 vorgesehen, von dem aus in der Art einer parallelen Medienzuführung in die Kathodenräume 42 der Elektrolysezellen 40 mündende Zuleitungen 50 abgehen. Ausgangsseitig der Kathodenräume 42 sind Abströmleitungen 52 vorgesehen, die in einem Sammelpunkt 54 zusammengeführt sind, so dass sich insgesamt eine medienseitig parallele Verschaltung der Kathodenräume 42 ergibt.

Anodenseitig ist hingegen eine Reihen- oder Hintereinanderschaltung der Anodenräume 44 für den Anolyten vorgesehen. Dazu ist der in Strömungsrichtung des Anolyten gesehen im ersten Elektrolysemodul 40 zugeordnete Anodenraum 44 ausgangsseitig über eine Überströmleitung 56 mit der Eingangsseite des nachfolgenden Anodenraums 44 verbunden. Dieser ist seinerseits ausgangsseitig an die Abströmleitung 24 angeschlossen, so dass sich in der Art einer mehrstufigen oder kaskadenartigen Ausführung eine Hintereinanderschaltung der Anodenräume 44 bzgl. des Anolyten ergibt.

Zudem ist in der Anlage 1 vorgesehen, den aus den Kathodenräumen 42 abströmenden Katholyten als Anolyt in die hintereinander geschalteten Anodenräume 44 einzuspeisen. Dazu ist der Sammelpunkt 54 für den Katholyten über eine Überströmleitung 58 mit dem Anodenraum 44 der in Strömungsrichtung des Anolyten gesehen ersten Elektrolysezelle 40 verbunden.

Von der Überströmleitung 58 zweigt im Sammelpunkt 54 zudem eine Abführleitung 60 ab, über die ein in seiner Menge über ein Drosselventil 62 einstellbarer Teilstrom des Katholyten einem Abwassersystem oder einem Auffangbehälter 64 zugeführt werden kann. Mit dieser Anordnung ist es möglich, eine einstellbare Teilmenge des aus den Kathodenräumen 42 abströmenden Katholyten als Anolyt der Kaskade aus Anodenräumen 44 zuzuführen. Damit können unter anderem die individuellen Reaktionsparameter wie beispielsweise Druck und Fließgeschwindigkeit in den Anodenräumen 44 geeignet beeinflusst werden, und zudem kann die Menge des in diesem Anwendungsfall als ,,Abfallprodukt" anfallenden Katholyts besonders gering gehalten werden.

Zur Überprüfung der Materialeigenschaften des hergestellten Anolyten sowie zur Mengenmessung sind im Übrigen in die Abströmleitung 24 eine Anzahl von Sensoren, insbesondere ein Mengensensor 66, ein Temperatursensor 68, ein pH-Sensor 70 sowie ein Sensor 72 zur Messung des Redoxpotentials, geschaltet.

Wie bereits erwähnt ist die Anlage 1 gemäß FIG. 1 gezielt für die Bereitstellung von besonders hochwertigem Anolyt durch kaskadenartige medienstromseitige Hintereinanderschaltung der Anodenräume 44 bei medienstromseitiger Parallelschaltung der Kathodenräume 42 ausgelegt. Im alternativen Ausführungsbeispiel gemäß FIG. 2 ist die Anlage 1' hingegen für die Bereitstellung von besonders angereichertem Katholyt durch kaskadenartige Hintereinanderschaltung der Kathodenräume 42 bei Parallelschaltung der Anodenräume 44 ausgelegt. Des-weiteren sind natürlich bedarfsweise auch kombinierte Verschaltungen dieser Grundkonzepte möglich.

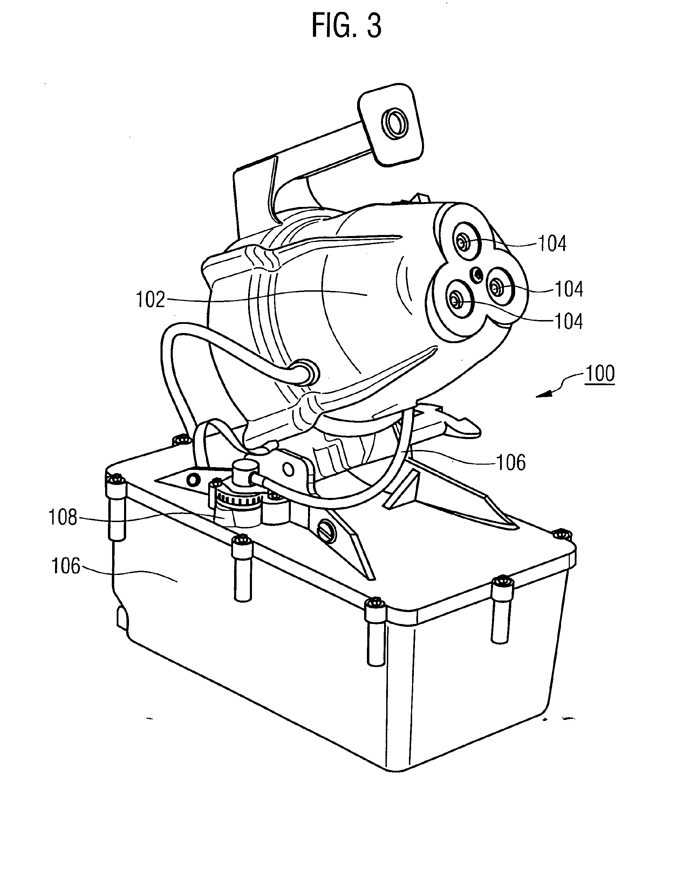

Die in der Anlage 1 erzeugte elektrochemich aktivierte Läsung wird nun als Desinfektionsmittel in einer Verneblungsvorrichtung 100 gemäß der FIG. 3 zur Raumdesinfektion verwendet. Die Verneblungsvorrichtung 100 ist dabei als tragbares Gerät ausgestaltet und umfasst eine Gebläsebaugruppe 102 mit drei Verneblungsdüsen 104, die jeweils aus Nylon gefertigt sind. Dadurch sind die Verneblungsdüsen 104 gegen Korrosion beständig. Unterhalb der Gebläsebaugruppe 102 ist der Desinfektionsmittelbehälter 106 angeordnet, der ein Dosierventil 108 sowie einen nicht näher gezeigten Filter aufweist.

Das innerhalb der Gebläsebaugruppe 102 angeordnete Gebläse ist ein zweistufiger Axialkompressor, der von einem Universalmotor angetrieben wird. Der Motor läuft mit einer Geschwindigkeit von 20000 U/Min. Das Gebläse fördert eine große Menge Luft durch das Düsensystem. Jede Verneblungsdüse 104 hat sechs feststehende Richtflügel, die die Luft beim Verlassen der Verneblungsdüse 104 in eine Drehbewegung versetzen. In der Mitte dieses Luftwirbels wird über den Versorgungsschlauch 110 das Desinfektionsmittel aus dem Desinfektionsmittelbehälter 106 eingeleitet, das durch den entstehenden Unterdruck aus dem Vorratstank gesaugt wird. Dadurch wird die Formulierung in kleinste Teilchen zerrissen und in die Umgebungsluft abgegeben.

Die Verneblungsvorrichtung 100 ist aus weitgehend säurebeständigem Polyethylen gefertigt und hat ein robustes, schlaggeschütztes Gehäuse. Über das Dosierventil 108 kann die Tröpfchengröße von 5 **-** 50 µm reguliert werden, was einer maximalen Durchflussleistung von 19 Liter in der Stunde entspricht. Durch den 4 Liter fassenden Dosiermittelbehälter 106 sowie den drei Präzisions-Verneblungsdüsen 104 für eine voluminöse Sprühmenge ist auch eine wirtschaftliche Behandlung größerer Objekte gewährleistet.

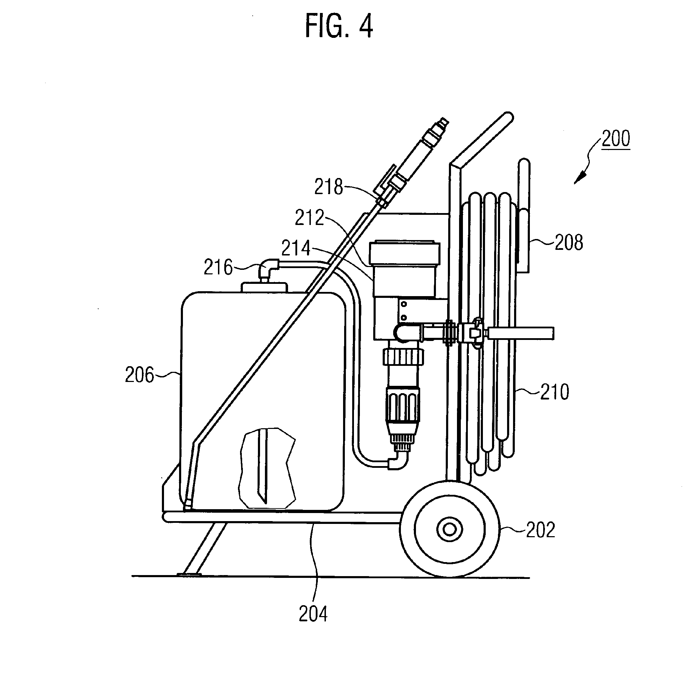

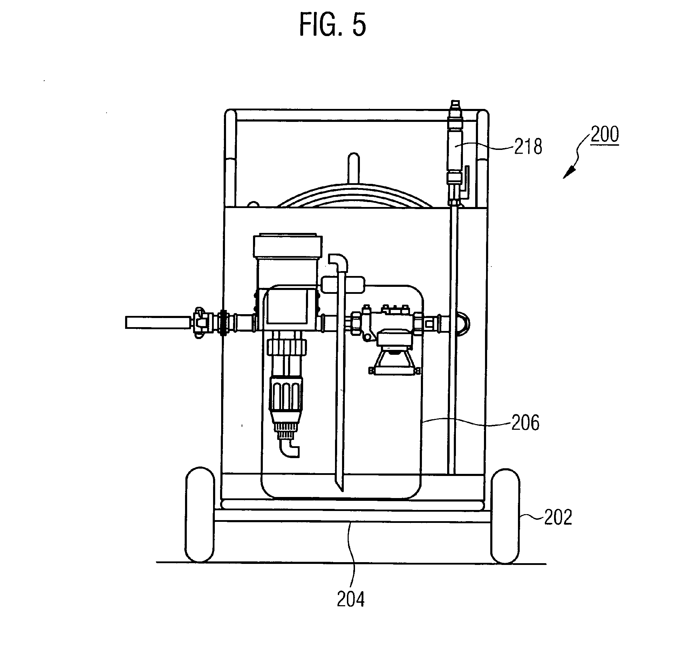

Ein zur Desinfektion von Oberflächen unter Verwendung des durch die Anlage 1 hergestellten elektrochemisch aktivierten Lösung als Konzentrat besonders geeignetes mobiles Austragsgerät 200 ist in den Figuren 4 und 5, jeweils in anderer Ansicht, dargestellt. Das mobile Austragsgerät 200 umfasst ein auf Rädern 202 laufendes Fahrwerk 204, auf dem in der Art eines Tragrahmes ein Vorratsbehälter 206 für das Konzentrat angeordnet ist. Des Weiteren trägt das Fahrwerk 204 auf einer Haltereinrichtung 208 einen Wasserschlauch 210, der eingangsseitig an ein Brauchwasser-Versorgungsnetz anschließbar ist und damit ein Anschlusssystem zur Verbindung mit dem Brauchwasser-Versorgungsnetz bildet. Der Wasserschlauch 210 ist an eine Dosiereinheit 212 ebenfalls angeordnet auf dem Fahrgestell 204, angeschlossen.

Die Dosiereinheit 212 umfasst einerseits eine Förderpumpe 214 und andererseits eine Venturidüse (in den Figuren 4, 5 innen liegend und daher nicht sichtbar), die über einen Überströmschlauch 216 mit dem Vorratsbehälter 206 verbunden ist. Ausgangsseitig ist die Förderpumpe 212 über einen Schlauch an eine als Austragseinheit für das Desinfektionsmittel vorgesehene Sprühlanze 218 angeschlossen.

Beim Betrieb des mobilen Austragsgeräts 200 wird über den Wasserschlauch 210 Brauchwasser aus dem Brauchwasser-Versorgungsnetz entnommen. Das Brauchwasser wird dabei von der Förderpumpe 214 zur Sprühlanze hin weiterbefördert. Durch die in der Dosiereinheit 212 vorgesehene Venturidüse wird dabei beim Durchströmen des Brauchwassers auf Grund des Venturieffekts Konzentrat aus dem Vorratsbehälter 206 angesaugt und dem Brauchwasser zugemischt. Die Zudosierung des Konzentrats zum Brauchwasser kann dabei durch den Förderdruck, also insbesondere den Wasserdruck im Brauchwasserstrang innerhalb des Austragsgeräts 200, und/oder über die Durchflussmenge geeignet eingestellt werden, wobei durch geeignete Einstellung nicht näher dargestellter Drosselventile Einspeiseraten beispielsweise im Bereich von 1 bis 10 % wählbar sind.

Das solchermaßen mit dem Konzentrat versetzte Brauchwasser wird von der Förderpumpe 214 als Desinfektionsmittel in die Sprühlanze 218 gefördert und kann von dieser aus auf die zu behandelnde Fläche aufgebracht werden.

### Bezugszeichenliste

- 1: Anlage
- 2: Elektrolysemodul
- 4: Versorgungsleitung
- 6: Wasserenthärterstation
- 8: Venturidüse
- 10: Solebehälter
- 12: Ablaufleitung
- 14: Soleeinspeiseleitung
- 16, 18: Ventil
- 20: Drosselventil
- 24: Abströmleitung
- 26: Vorratsbehälter
- 28: Multiwegeventil
- 30: Abflussleitung
- 40: Elektrolysezelle
- 42: Kathodenraum
- 44: Anodenraum
- 46: Membran
- 48: Verzweigungspunkt
- 50: Zuleitung
- 52: Abströmleitung
- 54: Sammelpunkt
- 56: Überströmleitung
- 58: Überströmleitung
- 60: Abführleitung
- 62: Drosselventil
- 64: Auffangbehälter
- 66: Mengensensor
- 68: Temperatursensor
- 70: pH-Sensor
- 72: Sensor
- 80: Hohlzylinder
- 82: Halterung
- 84: Öffnung
- 86: Membran
- 88: Hohlzylinder
- 90: Durchströmöffnung
- 100: Verneblungsvorrichtung
- 102: Gebläsebaugruppe
- 104: Verneblungsdüse
- 106: Desinfektionsmittelbehälter
- 108: Dosierventil
- 110: Versorgungsschlauch
- 200: Austragsgerät
- 202: Räder
- 204: Fahrwerk
- 206: Vorratsbehälter
- 208: Halteeinrichtung
- 210: Wasserschlauch
- 212: Dosiereinheit
- 214: Förderpumpe
- 216: Überströmschlauch
- 218: Sprühlanze

## Patentansprüche

1. Verfahren zur Desinfektion von Oberflächen, bei dem dezentral und in räumlicher Nähe zur zu behandelnden Oberfläche ein Desinfektionsmittel durch Zudosierung eines Konzentrats eines Desinfektionswirkstoffes zu einem Brauchwasserstrom erzeugt und über eine Austragseinheit auf die zu behandelnde Oberfläche aufgetragen wird.

2. Verfahren nach Anspruch 1, bei dem das Desinfektionsmittel zur Aufbringung auf die zu behandelnde Oberfläche in mikroskopische Tröpfchen vernebelt wird.

3. Verfahren nach Anspruch 1 oder 2, bei dem das Konzentrat zum Brauchwasserstrom über eine Venturidüse zudosiert wird.

4. Verfahren nach einem der Ansprüche 1 bis 3, bei dem der Anteil des in den Brauchwasserstrom zudosierten Konzentrats über den Wasserdruck und/oder die Durchflussmenge des Brauchwassers gesteuert wird.

5. Verfahren nach einem der Ansprüche 1 bis 4, bei dem als Konzentrat zur Erzeugung des Desinfektionsmittels eine elektrochemisch aktivierte Lösung verwendet wird, welche durch Elektrolyse von solehaltigem Wasser in einem eine Mehrzahl von Elektrolysezellen (40) umfassenden Elektrolysemodul (2) erzeugt ist, bei dem jede Elektrolysezelle (40) jeweils einen ersten Elektrodenraum und einen von diesem durch eine Membran (46) getrennten zweiten Elektrodenraum umfasst, wobei der Elektrolyt den ersten Elektrodenräumen der Elektrolysezellen (40) parallel und den zweiten Elektrodenräumen der Elektrolysezellen (40) in Reihenschaltung zugeführt wird.

6. Verfahren nach Anspruch 5, bei dem die ersten Elektrodenräume der Elektrolysezellen (40) jeweils als Kathodenräume (42) und die zweiten Elektrodenräume der Elektrolysezellen (40) jeweils als Anodenräume (44) ausgestaltet sind.

7. Verfahren nach Anspruch 5 oder 6, bei dem aus den ersten Elektrodenräumen der Elektrolysezellen (40) abströmender Elektrolyt dem zweiten Elektrodenraum der in Strömungsrichtung des Elektrolyten gesehen ersten Elektrolysezelle (40) zugeführt wird.

8. Verfahren nach einem der Ansprüche 5 bis 7, bei dem lediglich ein Teilstrom des aus den ersten Elektrodenräumen der Elektrolysezellen (40) abströmenden Elektrolyts dem zweiten Elektrodenraum der in Strömungsrichtung des Elektrolyten gesehen ersten Elektrolysezelle (40) zugeführt wird.

9. Verfahren nach einem der Ansprüche 5 bis 8, bei dem den ersten Elektrodenräumen Sole aus einem Solebehälter (10) über eine Venturidüse (8) zugeführt wird.

10. Verfahren nach einem der vorhergehenden Ansprüche, bei dem mikroskopische Tröpfchen mit einer Größe von 5-50 Mikron erzeugt werden.

11. Vernebelungsvorrichtung (100) mit einem Desinfektionsmittelbehälter (106) und einer Vernebelungsdüse (104), welche das Verfahren nach einem der vorhergehenden Ansprüche ausführt.

12. Vernebelungsvorrichtung (100) nach Anspruch 10, bei der die Vernebelungsdüse (104) aus einem Kunststoff gefertigt ist.

13. Mobiles Austragsgerät (200) zur Ausbringung eines Desinfektionsmittels auf eine zu behandelnde Oberfläche, mit einem Vorratsbehälter für ein Konzentrat eines Desinfektionswirkstoffes, mit einem Anschlusssystem zur Verbindung mit einem Brauchwasser-Versorgungsnetz, und mit einer ausgangsseitig mit einer Austragseinheit und eingangsseitig sowohl mit dem Vorratsbehälter als auch mit dem Anschlusssystem verbundenen Dosiereinheit.

14. Mobiles Austragsgerät (200) nach Anspruch 13, dessen Dosiereinheit eine Venturidüse umfasst.

15. Mobiles Austragsgerät (200) nach Anspruch 13 oder 14, dessen Dosiereinheit eine hinsichtlich ihrer Fördermenge einstellbare Förderpumpe umfasst.

16. Mobiles Austragsgerät (20) nach einem der Ansprüche 13 bis 15, dessen Austragseinheit als Sprühlanze ausgstaltet ist.

17. Mobiles Austragsgerät (200) nach einem der Ansprüche 13 bis 16, dessen Austragseinheit eine Vernebelungseinheit (100) nach Anspruch 11 oder 12 umfasst.
